**Europäisches Patentamt**

**European Patent Office**

**Office européen des brevets**

(19)

(11) Veröffentlichungsnummer: **0 237 817**

**A2**

(12) ## EUROPÄISCHE PATENTANMELDUNG

(21) Anmeldenummer: **87102297.6**

(51) Int. Cl.⁴: **G01N 30/60**

(22) Anmeldetag: **18.02.87**

(30) Priorität: **17.03.86 DE 3608883**

(43) Veröffentlichungstag der Anmeldung:
**23.09.87 Patentblatt 87/39**

(84) Benannte Vertragsstaaten:
**AT BE CH DE FR GB IT LI SE**

(71) Anmelder: **Gilak, Armin, Dr.**
**Grenzstrasse 8**
**D-5309 Meckenheim-Merl(DE)**

(72) Erfinder: **Gilak, Armin, Dr.**
**Grenzstrasse 8**
**D-5309 Meckenheim-Merl(DE)**

(74) Vertreter: **Patentanwälte Müller-Boré, Deufel,**
**Schön, Hertel, Lewald, Otto**
**Postfach 26 02 47 Isartorplatz 6**
**D-8000 München 26(DE)**

(54) **Chromatographische Säule für immunologische Untersuchungsverfahren.**

(57) Die Erfindung betrifft eine chromatographische Säule zum Separieren der Antigen-Antikörper-Komplexe von den freien Antigenen, wobei in einer wasserfesten und wasserdichten Umhüllung als unpolares Säulenmaterial ein kreppförmiges dicht gerolltes Filterpapier mit hohem Reinheitsgrad, insbesondere aus Regeneratzellulose, eingepreßt ist.

Erfindungsgemäß weist die chromatographische stehende Säule einen oberen von oben beaufschlagbaren Reaktionsteil 13 und einen unteren Trenn-und Meßteil 10; 32 auf, die durch einen perforierbaren Boden 16; 22 voneinander getrennt sind.

EP 0 237 817 A2

FIG. 1

## Chromatographische Säule für immunologische Untersuchungsverfahren

Die Erfindung betrifft eine chromatographische Säule für immunologische Untersuchungsverfahren.

Mit einer solchen chromatographischen Säule sollen Separationsverfahren für immunologische Bestimmungen, insbesondere fluoreszenz-, enzym- oder lumineszenz-immunologische Untersuchungen durchgeführt werden, bei denen der gebildete Antigen-Antikörper-Komplex von den freien, nicht am Antikörper gebundenen Antigenen mit Hilfe der trockenen chromatographischen Säule getrennt wird, welcher das flüssige Reaktionsgemisch der immunologischen Bestimmung aufgegeven wird und wobei die Antigen-Antikörper-Komplex-Lösung extinktionphotometrisch gemessen wird.

Der Erfindung liegt die Aufgabe zugrunde, eine besonders preiswerte und einfach zu handhabende chromatographische Säule für solche Untersuchungsverfahren vorzuschlagen, die in besonders einfacher und zuverlässiger Weise Reaktionen, Trennung und Messung der zu untersuchenden Substanzen ermöglicht. Demgegenüber wird erfindungsgemäß die oben genannte Aufgabe bei einer trockenen chromatographischen Säule zum Separieren der Antigen-Antikörper-Komplexe von den freien, nicht an Antikörper gebundenen Antigenen und/oder zur vollständigen Bindung aller markierten Substanzen bei der immunologischen Bestimmung von Antigenen bzw. Haptenen nach radio-,fluoreszenz-,lumineszenz oder enzym-immunologischen Bestimmungsmethoden, bei der in einer wasserfesten und wasserdichten Umhüllung als unpolares Säulenmaterial ein kreppförmiges dichtgerolltes Filterpapier mit hohem Reinheitsgrad, insbesondere aus Regeneratzellulose eingepreßt ist, dadurch erreicht, daß die chromatographische stehende Säule einen oberen Reaktionsteil und einen unteren Trenn-und Meßteil aufweist, die durch einen perforierbaren Boden voneinander getrennt sind.

Hierdurch wird erreicht, daß Reaktion einerseits, Trennung und Messung andererseits, in einer einzigen Säule und zwar gegebenenfalls ohne äußeren Kontakt durchgeführt werden können, das zu untersuchende Material, insbesondere das radioaktive Material, vollständig im Filter verbleibt und in einfachster Weise mittels eines Gamma-Counters bei radioimmunologischen Untersuchungen bzw. photometrisch bei lumineszenz-immunologischen Untersuchungen gemessen werden kann.

Als ein wichtiger erwünschter Nebeneffekt bei den radioimmunologischen Bestimmungen mit der erfindungsgemäßen Säule ist anzusehen, daß bei radioimmunologischen Bestimmungen das gesamte radioaktive Reaktionsgemisch in der Säule aufgesaugt verbleibt. Nach der Auszählung der Problem liegt somit die Radiokativität in einer bequemen handhabbaren Form vor; die Gefahr einer Kontaminierung mit Resten des flüssigen Reaktionsgemisches ist gegenüber den üblichen Verfahren stark gesenkt. Auch wenn der Antikörper stationär an eine Trägersubstanz innerhalb des Proberöhrchens gebunden ist, wird die gesamte flüssige Radioaktivität in der Säule absorbiert; dabei werden gleichzeitig die freien Antigene im oberen Säulenabschnitt gebunden.

Vorzugsweise wird die Filterpapiersäule von unten dicht an den perforierbaren Boden des oben aufschlagbaren Reaktionsteils herangesteckt. Hierdurch wird beim Perforieren bis in die Tiefe des Filterpapiers ein besonders wirksames Eindringen und ein ebensolches Adsorbieren erreicht. Zweckmäßig kann die Filterpapiersäule obeneine trichterartige Aufweitung haben, mit der sie im Klemmsitz unter den formähnlichen Boden der Säule gesteckt ist.

Das Filterpapier kann in eine Kunststoffhülse eingepreßt sein, die gegebenenfalls von einer metallischen Hülse, insbesondere aus verzinktem Kupfer, umgeben ist. Diese eignet sich als Abschirmmaterial bei extinktionsphotometrischen bzw. radioimmunologischen Messungen. Die Perforierung kann von außen vorgenommen werden. Es ist aber auch möglich, mit dem Boden in Berührung stehende Teile mit Zacken, Spitzen oder dergleichen auszustatten, die, etwa bei Druck auf die beiden Enden der Säule dann den Boden perforieren (geschlossenes System). Eine besondere Anwendung von Säule und Filter auf Automaten ist erfindungsgemäß ebenfalls in Betracht gezogen. Hierbei werden zwei unten geschlossene röhrenartige Mikroküvetten nebeneinander angeordnet, wobei die erste als Reaktionsgefäß mit perforierbarem Deckel dient. Die zweite dient als Trenn-und Meßgefäß und enthält im oberen Abschnitt dicht eingepaßt in eine Kunststoffhülse das säulenartige Filterpapier, im unteren Abschnitt erfolgt die extinktionsphotometrische Messung. Statt der Verwendung von 2 Mikroküvetten ist auch eine einzige Vorrichtung denkbar, wobei oben der beschriebene Reaktionsteil mit perforierbarem Boden und unten der Trenn-und Meßteil angeordnet sind. Der Trennteil besteht aus der oben beschriebenen Filterpapiersäule, oben trichterförmig aufgeweitet und im Klemmsitz unter den Boden des Reaktionsteils gesteckt. Der Meßteil ist eine Mikroküvette, die von unten über die Kunststoffhülse des Reaktionsteils geschoben ist.

Bei dieser Ausführungsform ist es möglich, die Reagenzien mit Ausnahme der zu bestimmenden Substanz bei tiefen Temperaturen, insbesondere unterhalb -20°C, zu lyophilisieren.

Schließlich ist bei Einsatz der Maßnahme nach der Erfindung in Automaten möglich, alle Säulen durch einen einzigen Beaufschlagungsvorgang, manuell oder apparativ, perforierbar auszubilden. Die Beaufschlagung der Säule erfolgt, wie bei allen Weiterbildungen der Erfindung, von oben.

Eine besonders zweckmäßige wichtige Weiterbildung der Erfindung ist darin zu sehen, das kreppförmige Filtermaterial zur Erhöhung seiner Bindungsaffinität mit einer organischen Säure, insbesondere Malein-oder Oxalsäure oder gegebenenfalls einer Base, zu imprägnieren, und das Imprägniermittel wieder auszuwaschen. Dies verleiht dem wiedergetrockneten Papier durch eine gewisse Quellung der Fasern eine verbesserte Absorptionsfähigkeit und Differenzierung der Absorption.

Beispielsweise Ausführungsformen sollen nun mit Bezug auf die beiliegenden Zeichnungen näher erläutert werden, in denen

Fig. 1 eine chromatographische Säule für das radioimmunologische Untersuchungsverfahren (Radio-Immuno-Assay•RIA);

Fig. 2 eine chromatographische Säule z.B. für fluoreszenz-oder lumineszenz-immunologische Untersuchungsmethoden;

Fig. 3 eine Ausführungsform mit Mikroküvette;

Fig. 4/5 Reaktionsgefäß und Meßgefäß für Automaten und

Fig. 6 eine weitere Ausführungsform zeigt.

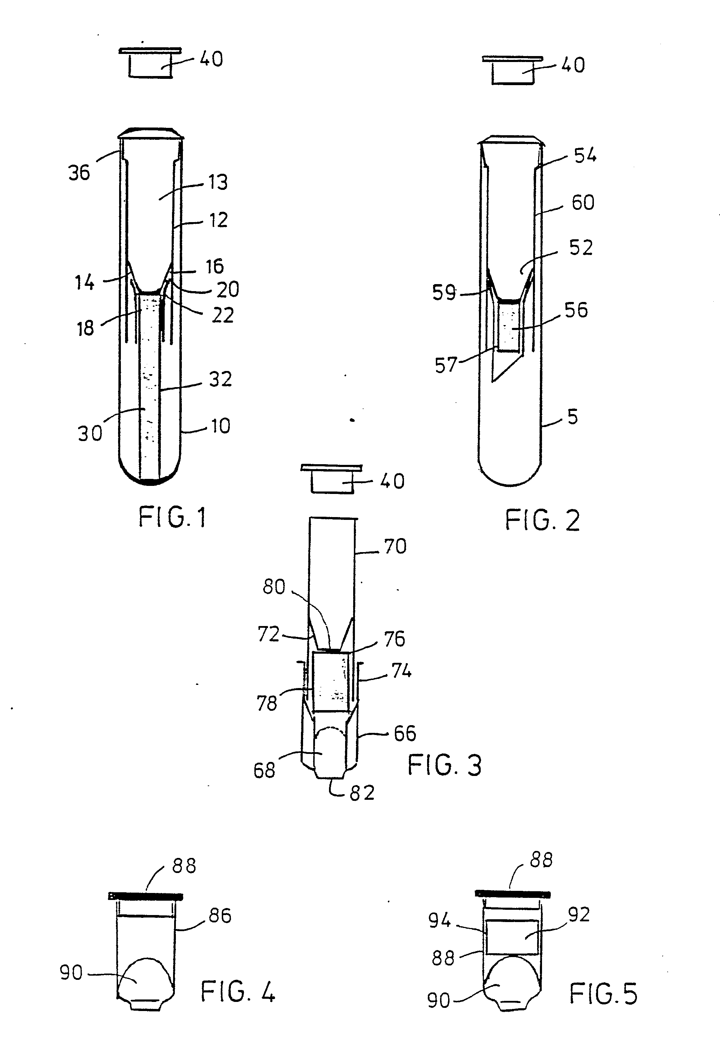

Fig. 1 zeigt für radioimmunologische Bestimmungen ein Probenröhrchen 10, in das eine stehende chromatographische Säule 12 dicht eingeschoben ist, die etwa in der Mitte über einen Boden 14 verfügt. Die Säule mit Ausnahme des Filterpapiers und das Probenröhrchen bestehen aus einem durchsichtigen Kunststoff, vorzugsweise aus Polyethylen. Der Boden 14 des Reaktionsgefäßes ist fest und abgedichtet bezüglich der Säule angebracht. Der Boden 14 ist bei 16 konisch oder trichterförmig nach unten ausgebildet und verfügt über eine dünne perforierbare Basis 18.

Durch die Konizität des Trichterbodens entsteht ein entsprechender Ringraum 20, der aus der Innenwand des geraden Säulenrohres und der Außenwand des trichterartigen Bodens gebildet ist und oben spitz zuläuft. Diese einfache Konstruktion wird ausgenützt, um in unten zu beschreibender Weise eine Klemmverbindung zu ermöglichen. Komplementär zu diesem Trichterboden verfügt der Filter-und Meßteil des Trenngefäßes über eine Hülse, die in einen komplementär trichterförmig aufgeweiteten Teil 22 übergeht. Die Hülse kann aus Metall, aber auch aus Kunststoff bestehen. In die Hülse ist dicht eingepreßt ein Filter 30 aus präpariertem Krepp-Papier, welches das eigentliche Säulenmaterial bildet. Es verfügt über eine Umhüllung 32 aus einem äußeren steifen Kunststoff. Das Säulenmaterial kann aus Zellulose bestehen, die mit chemischen Imprägnierungsmittelt z.B. mit organischen Säuren, vorzugsweise Malein-oder Oxalsäure oder ggf. mit einer Base, vorbehandelt wurde. Das mit Hülse versehene Reaktionsröhrchen wird in den Trichteransatz der Säule so eingesteckt, daß das Filter 30 den perforierbaren Boden kontaktiert. Bei der erfindungsgemäßen Durchführung der Probenbestimmung werden die Reagenzien in den Reaktionsteil 13 pipettiert. Nach Einstellung des Reaktionsgleichgewichtes am Ende der Inkubationszeit wird mit einem spitzen Gegenstand der trichterförmige Boden des Reaktionsteils von unten oder, bevorzugt von oben, perforiert und zwar ein gutes Stück in das Kreppfilter hinein. Das flüssige Reaktionsgemisch läuft durch den perforierten Boden in den säulenförmigen Filter. Dabei erfolgt die Trennung in der Form, daß die ungebundene Phase, d.h. die freien, nicht an Antikörper gebundenen Antigene im oberen Säulenabschnitt absorbiert werden, während die gebundene Phase, d.h. der Antigen/Antikörper-Komplex in den unteren Säulenabschnitt wandert. Sämtliche radioaktive Flüssigkeit wird von dem wasserdicht umhüllten Filterpapier aufgesogen und kann somit ohne Kontaminationsgefahr leicht entsorgt werden. Bei der Testdurchführung kann selbstverständlich das Röhrchen mit einem Deckel 40 verschlossen werden. Durch das enge Anliegen des Filtermaterials am Boden des Reaktionsgefäßes und das durchgehende Durchstechen ist eine ausgezeichnete Benetzung des gesamten Säulenmaterials 30 gewährleistet. Andere Möglichkeiten der senkrechten Fixierung des Filterpapiers mit Hülse innerhalb der gesamten Säule sind selbstverständlich möglich, wie kleine Rippen an dem einen Teil, Ausnehmungen am anderen Teil, ein Ringwulst am trichterförmigen Boden und eine Ringnut an der aufgeweiteten Hülse etc.. Nachzutragen ist noch, daß der obere Säulenabschnitt, das Reaktionsgefäß 13, über einen aufgeweiteten Teil 36 (Passkragen) in das Hüllrohr 10 eingepaßt ist.

Fig. 2 zeigt ein Ausführungsbeispiel, insbesondere für lumineszenz-, fluoreszenz-und enzymimmunologische Untersuchungsverfahren. In einem Hüllrohr 50 wird eine ähnliche Konstruktion wie in Fig. 1 in Form einer Säule 52 mit satt gegen die Hüllrohrinnenseite einpassenden Säulenkopf 54 eingeschoben.

Handelte es sich bei der Konstruktion der Fig. 1 im wesentlichen bei der Hülsenaufweitung des langen Filters um Metall in relativ kurzer Ausbildung der Aufweitung, so ist für das Lumineszenzverfahren nur ein kurzes Stück Filter 56, gegebenenfalls mit Kunststoffaußenhülle, in einem beispielsweise langen metallischen Hülsenrohr 57 gehalten. Eine beispielsweise aus Kunststoff bestehende aufgeweitete Manschette 59 ist in ähnlicher Weise wie in Fig. 1 über den komplementären Trichterboden 58 der Säule gesteckt. Diesmal geht die aufgeweitete Hülse/Abschirmung 59 bis ganz oben an den zwischen der Innenseite des geraden Säulenrohrs und der Außenseite des trichterförmigen Bodens 58 gebildeten Zwischen-oder Ringraum und ist satt in diesem bei geringem Andruck enthalten. Auch das gegebenenfalls metallische und aus verzinktem Kupfer bestehende Hülsenrohr 57 für die Fixierung des Filters dient als Abschirmung. Nach der Darstellung ist die Säule unten schräg zugeschnitten, um ein Abtropfen der nur in geringen Mengen gegebenenfalls vorhandenen Flüssigkeit zwangsweise sicherzustellen. Ausführungsformen ohne Abschirmung 59 sind möglich. Das Filterpapier kann auch in die Kapillarhülle eingepreßt werden. Zur fluoreszenz-immunologischen Untersuchung werden die Reagenzien in den Reaktionsteil 60 der chromatographischen Säule einschließlich der markierenden Substanz pipettiert. Nach Einstellung des Reaktionsgleichgewichts wird der trichterförmige Boden des Reaktionsteils bis an das dicht darunter liegende Filterpapier perforiert und das flüssige Reaktionsgemisch fließt von oben in die Filtersäule. Dabei werden die Komponenten getrennt, indem die freie Phase im oberen Säulenabschnitt gebunden wird und die gebundene Phase, d.h. der zu messende Antigen/Antiköroper/Komplex die Trennsäule durchläuft und auf den Boden des Probenröhrchens hinunter tropft und hier also extinktionsphotometrisch gemessen werden kann.

Die Abschirmung 59 kann eingefärbt sein.

Fig. 3 zeigt eine Ausführungsform mit einer Meßküvette 66, die als Mikroküvette ausgebildet ist und, um auch für sehr kleine Mengen abgefilterer Flüssigkeit brauchbar zu sein, im unteren Bereich 68 eingeschnürt ist. Es ist interessant, daß die gleiche Säule 70 mit praktisch dem gleichen trichterartigen Boden 72, wie bezüglich Fig. 1 und 2 beschrieben, in diese Meßküvette paßt und einfach mit ihrem unteren offenen Ende 74 eingeschoben wird. In diesem Fall ist das Filtermaterial 76 eingepreßt in einer Kunststoffhülle 78, die dicht dem perforierbarem Boden 80 des trichterförmigen Reaktionsgefäßes anliegt. Das Reaktionsgefäß kann wieder durch einen Deckel 40 abgeschlossen sein. Ähnlich wie in Fig. 1 das untere Ende des Hüllrohres bildet hier die Mikroküvette das Meßgefäß, die Säule 70 das Reaktions-und Trenngefäß. Der Antigen/Antikörper/Komplex kann wieder photometrisch gemessen werden. In diesem Fall ist keine gesonderte Abschirmung vorgesehen, kann aber angeordnet werden. Es handelt sich bei der Mikroküvette um eine Konstruktion mit aus meßtechnischen Gründen besonders kleinem Boden 82.

Besonders für Automaten sind die Mikroküvetten 86 und 88 der Figuren 4 und 5 geeignet. Die Mikroküvette 86 bildet das Reaktionsgefäß, die Mikroküvette 88 der Fig. 5 das Meßgefäß. Die Mikroküvetten können beispielsweise gruppiert in Racks angeordnet werden. Bei der Messung wird das Reaktionsgefäß dann immer übergangen und nur im Meßgefäß gemessen. Die beiden Gefäße sind durch dichtschließende steckbare Deckel 88 abgeschlossen. Die Mikroküvetten sind unten wieder eingeschnürt, um einmal gut in den Halter des Automaten zu passen, zum anderen, um auch kleinste definierte Volumina messen zu können, wie bei 90 zu sehen ist. Bei der Probenuntersuchung nach immunologischen Verfahren, besonders bei fluoreszenz-immunologischen Bestimmungen erfolgt nach bestimmtem Ablauf das Pipettieren der Reagenzien in die Mikroküvette 86 als Reaktionsgefäß. Nach Be endigung der Inkubationszeit wird das Reaktionsgemisch aspiriert und in der Meßküvette 88 auf den Filter gegeben. Wenn die Flüssigkeit den säulenförmigen Filter durchläuft, werden die Komponenten getrennt wie für Fig. 2 oder 3 beschrieben. Die Flüssigkeit auf dem Boden dere Meßküvette enthält den Antigen/Antikörper/Komplex und kann photometrisch gemessen werden. Die Mikroküvette 88 kann die gleiche Form wie die Mikroküvette 86 der Fig. 4 haben. Beim Filter kann es sich um das gleiche Filtermaterial wie schon beschrieben und unten näher erläutert handeln. Das Filtermaterial wird hier in eine den Innenumfang der Mikroküvette 88 voll ausfüllende beispielsweise aus Kunststoff bestehende Hülse 94 gegeben.

Durch die Maßnahme nach der Erfindung erfolgt also die Reagenzienaufgabe oben in der beschriebenen chromatographischen Säule, im Mittelteil wird gefiltert und getrennt, unten wird gemessen.

Beim Säulenmaterial handelt es sich um ein trockenes Adsorptionsmittel aus unpolarem Material in Form eines homogenen verfilzten kreppförmigen und zu einer dichten Säule gerollten Filterpapiers mit hohem Reinheitsgrad. Das Filterpapier besteht aus reinem Linters mit einem Polymerisationsgrad von 2000 -3000 oder aus Regeneratzellulose mit einem Polymerisierungsgrad von 800 -3000 und ist frei von löslichen Stoffen. Das verwendete Filterpapier besitzt eine gleichmäßige Textur mit Poren in der Größenordnung von 1 -14

μm und weist über der Höhe der Säule eine gleichmäßige Saugfähigkeit auf. Um die Bindungsaffinität zu erhöhen, ist das Filterpapier mit Säuren, vorzugsweise Malein-oder Oxalsäure, ggf. auch mit Basen behandelt. Die Trennung erfolgt - schnell und äußerst präzise durch das Zusammenwirken von Schwerkraft und Kapillarkräften. Die Auftrennung erfolgt überraschenderweise so, daß die niedermolekularen Bestandteile (freie Antigene und Antikörper) im oberen Säulenabschnitt, d.h. nach kurzer Laufzeit, gebunden werden, während die hochmolekularen Antigen/Antikörper/Komplexe ungebunden die Säule durchwandern. In Fig. 1, bei unten abgeschlossener Säule, verbleiben die Komplexe im unteren Säulenabschnitt. In Fig. 2 mit unten offener Säule ist der Komplex in der nach unten abtropfenden Flüssigkeit enthalten. Vermutlich überwiegen bei dem verwendeten umpolaren Adsorptionsmittel van der Waals' und hydrophobe Wechselwirkungen, die das Wanderungsverhalten erklären können.

Bei allen Ausführungsformen können sämtliche Reagenzien mit Ausnahme der zu bestimmenden Substanz in der Säule lyophilisiert werden.

Die Ausführungsform nach Fig. 1 für radio-immunologische Bestimmungen enthält eine Metallabschirmung im oberen Filterabschnitt, so daß nur die Radioaktivität des unten befindlichen Antigen/Antikörper/Komplexes gemessen wird. Ähnlich enthalten die Ausführungsformen für fluoreszenz-oder lumineszenz-immunologische Untersuchungen (Fig. 2, 3, 5) eine Abschirmung aus Kunststoff um das säulenartige Filterpapier zur Vermeidung jeder Lichtreflexion.

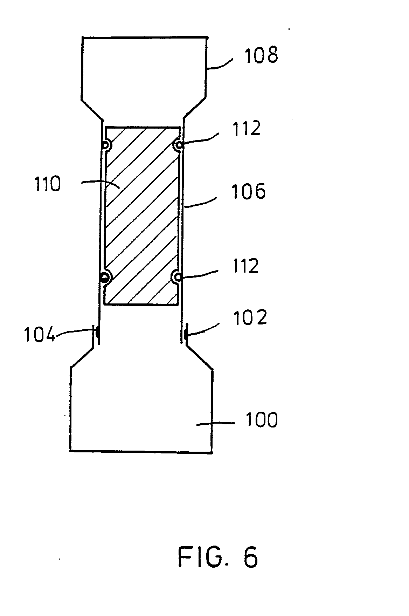

Eine andere Möglichkeit, ein Verfahren gemäß der Erfindung durchzuführen, besteht darin (Fig. 6), mit einem Reaktionsgefäß 100 aus einem Material, wie vorher erwähnt, gegebenenfalls nach Aufsatz einer nicht dargestellten Kappe, zu arbeiten. Nach der Reaktion im Gefäß 100 und nach Zugabe der markierenden Substanzen wird der Filter-und Tennteil 106 dicht an den Hals 102 des Reaktionsgefäßes gesteckt. Dichtungen 104 sind hierzu angedeutet. Es kann sich aber auch um jede andere formschlüssige gut abdichtende Verbindung handeln. In dem Trenn-und Meßteil 106 ist ein Filter 110 der vorbeschriebenen Art dicht eingepreßt. Die Pressung kann über nur angedeutete Rippen 112, die oben und unten vorgesehen sein können, vergrößert werden. Der Meß-und Trennteil ist hierbei durchgehend, d.h. es fällt der vorher erwähnte perforierbare Boden fort. Nach dem Aufstecken wird das Ganze um 180° gedreht (auf den Kopf gestellt). Die Lösung läuft durch das in oben beschriebener Weise vorpräparierte Filter durch. Die Messung erfolgt in vorbeschriebener Weise. Das Trennen von Kopf-und Bodenteil kann natürlich an einer anderen Stelle, beispielsweise nahe dem unteren Teil des Reaktionsgefäßes 108 oder an einer anderen zweckmäßigen Stelle vorgenommen werden. Die Bereitstellung erfolgt vorzugsweise als Kit, enthaltend die chromatographische Säule sowie die üblichen bekannten Reagentien für ein bestimmtes Testverfahren, gegebenenfalls in abgemessenen Einheitsmengen.

Das Meßgerät kann hierbei auch wie die Mikroküvette der Fig. 5 (ohne zu perforierenden Boden) geformt und mit dem Reaktionsgefäß zusammensteckbar ausgebildet sein.

Schließlich ist es, beispielsweise für Automaten, noch möglich, etwa durch gleichzeitiges Aufdrücken eine ganze Serie von Säulen zu perforieren. Dies kann beispielsweise von Hand oder über eine Platte geschehen. Hierzu könnte beispielsweise der Deckel mit langen spitzen Zacken versehen sein. Die Perforation würde bei Druck auf den Deckel erfolgen. Hier wäre es allerdings notwendig, die "Säule" ausreichend steif auszubilden. Der trichterförmige Boden der Säule wird im wesentlichen vorgeschwächt ausgebildet.

## Ansprüche

1. Chromatographische Säule zum Separieren der Antigen-Antikörper-Komplexe von den freien, nicht an Antikörper gebundenen Antigenen und/oder zur vollständigen Bindung aller markierten Substanzen bei der immunologischen Bestimmung von Antigenen bzw. Haptenen nach radio-,lumineszens-fluoreszenz-oder enzym-immunologischen Bestimmungsmethoden, bei der in einer wasserfesten und wasserdichten Umhüllung als unpolares Säulenmaterial ein kreppförmiges dichtgerolltes Filterpapier mit hohem Reinheitsgrad, insbesondere aus Regeneratzellulose, eingepreßt ist, dadurch **gekennzeichnet,** daß die chromatographische stehende Säule aus Filterpapier einen oberen von oben beaufschlagbaren Reaktionsteil (13) und einen unteren Trenn-und Meßteil (10; 32 bzw. 50; 56 bzw. 76; 78) aufweist, die durch einen perforierbaren Boden (16; 22 bzw. 58 bzw. 80) voneinander getrennt sind, wobei die Säule von unten dicht an den perforierbaren Boden der Säule herangesteckt ist.

2. Chromatographische Säule nach Anspruch 1, dadurch **gekennzeichnet,** daß die Säule oben und außen eine trichterartige Aufweitung (16;58;72) trägt, mit der sie in Klemmsitz unter den in der Form ähnlichen Boden der Säule gesteckt ist.

3. Chromatographische Säule nach einem der vorhergehenden Ansprüche, dadurch **gekennzeichnet,** daß die Säule aus Filtermaterial in eine Kunststoffhülse eingepreßt ist, die gegebenenfalls von einer metallischen Hülse, insbesondere aus verzinktem Kupfer, umgeben ist.

4. Chromatographische Säule nach einem der Ansprüche 1 bis 2, dadurch **gekennzeichnet**, daß der Filtermaterialteil der Säule von einem Material, insbesondere Kunststoff, als Paßteil zur Säuleninnenwand umhüllt ist, mit dem sie gegen den konischen bzw. trichterförmigen Boden der Säule geklemmt ist.

5. Chromatographische Säule nach einem der vorhergehenden Ansprüche, dadurch **gekennzeichnet**, daß die Säule insgesamt mittels eines Paßkragens (36) an ihrem oberen Ende in ein Kunststoffreagenzröhrchen dicht gesteckt ist.

6. Chromatographische Säule nach einem der vorhergehenden Ansprüche, dadurch **gekennzeichnet**, daß der Filterteil der Säule insbesondere für photometrische Messungen, gegen den Boden des Reaktionsteils abgeschirmt ist, wobei die trichterartig aufgeweitete Abschirmung aus einem perforierbaren, insbesondere nach unten trichterartigen Boden besteht.

7. Chromatographische Säule nach einem der Ansprüche 2 bis 6, dadurch **gekennzeichnet**, daß die trichterartig aufgeweitere Abschirmung oben an dem ansteckbaren Filterteil mit den Boden von unten perforierenden scharfen Vorsprüngen ausgebildet ist.

8. Chromatographische Säule nach einem der vorhergehenden Ansprüche, dadurch **gekennzeichnet**, daß der Filtermaterialteil der Säule zur Erhöhung seiner Bindungsaffinität durch chemische Imprägnierungsmittel wie organische Säuren, insbesondere Maleinsäure oder Oxalsäure oder ggf. einer Base, präpariert ist.

9. Abänderung der chromatographischen Säule nach Anspruch 1, **gekennzeichnet** durch einen unteren Reaktionsbehälter (100) mit aufsteckbarem Filter-und Meßteil (106), in welchem sich, dicht gepreßt, ein die Wandung dicht abschließendes, gegebenenfalls in einer Hülle sitzendes zur Erhöhung seiner Bindungsaffinität vorpräpariertes Kreppfiltermaterial (110) befindet, wobei die Säule für den Trenn-und Meßvorgang um 180° (auf den Kopf) verdrehbar bzw. stellbar ist.

10. Anwendung der chromatographischen Säule nach den Ansprüchen 1 bis 9 auf Automaten, dadurch **gekennzeichnet**, daß zwei unten geschlossene röhrenartige Mikroküvetten nebeneinander angeordnet sind, daß ein als Reaktionsgefäß dienender Säulenaufsatz oder Ausbildung der Mikroküvette selbst als Reaktionsgefäß bei perforierbarem Deckel
und auf der zweiten Mikroküvette ein Trenn-oder Meßsäulenaufsatz mit Säule aus Filterpapier oder Einsatz des Filters allein in die zweite Mikroküvette, wobei Lumineszenzmessungen ausschließlich jeweils an der zweiten Mikroküvette erfolgen.

FIG.1

FIG. 2

FIG. 3

FIG. 4

FIG.5

FIG. 6